# EUROPEAN PATENT SPECIFICATION

(11) **EP 2 617 463 B1**
(45) Date of publication and mention of the grant of the patent: **07.10.2015**
(21) Application number: 12000283.7
(22) Date of filing: 18.01.2012
(51) Int. Cl.: A61N 5/10

(54) **Radiotherapy apparatus**
Strahlentherapievorrichtung
Appareil de radiothérapie

(43) Date of publication of application: 24.07.2013
(73) Proprietor: Elekta AB (publ), 103 93 Stockholm (SE)
(72) Inventor: Carlsson, Per, Stockholm 103 93 (SE)
(74) Representative: Downing, Michael Philip

(56) References cited:
- WO-A2-2009/055775

## Description

### FIELD OF THE INVENTION

The present invention relates to radiotherapy,

### BACKGROUND ART

Radiotherapy consists of directing beams of Ionizing radiation toward a tumour. The radiation (usually high-energy x-rays) is absorbed by the tumour cells causing cell damage. Obviously, the radiation Is also capable of damaging the healthy tissue around the tumour, so various techniques are used in order to limit this, The dose is usually delivered in time-separated fractions (i.e. individual treatment sessions), allowing the healthy tissue to recover between fractions, and the doses are carefully collimated so that the amount of healthy tissue that Is irradiated Is minimised.

Within a single fraction, the dose may be delivered from a number of different directions so that the dose to each region of healthy tissue is reduced, This can be delivered In discrete stages, with the radiation source being repositioned after each stage, or the source can be moved continuously around the patient during delivery of the dose.

Where a beam of radiation is being collimated to a specific area, that area will of course be one that is defined relative to the anatomy of the patient. Therefore, the location of the patient needs to be established in advance of treatment so that the beam can be appropriately directed. Usually, a CT scan of the patient (by which we mean to include both a fan- or pencil-beam slice-by-slice CT scan or a CBCT scan, or "cone-beam CT" scan) will be taken immediately prior to each fraction of a fractionated treatment, while the patient Is on the couch ready for treatment, This will identify the patient's exact location with accuracy. Modern radiotherapy machines Include cone-beam CT scanning capability to permit this.

The international patent application WO 2009/055775 A2 discloses a radiotherapy planning apparatus wherein a treatment plan is adapted according to the proximity of the target volume to an organ at risk by varying the fraction size given on any individual day, based on the use of daily patient registration,

### SUMMARY OF THE INVENTION

A cone-beam CT scan at the start of a fraction deals with inter-fraction movement of a patient (i,e movement that takes place between the end of one fraction, or treatment session, and the start of the next fraction). However, it does not prevent or reveal intra-fraction movement (i.e. movement that takes place during the delivery of a single treatment fractlon). Patients can be asked to lie still, but are generally only able to do so with the accuracy required for a limited period of time. The patient can be restrained, but this is uncomfortable and only particularly effective for regions such as the head and upper neck where there is a hard structure (the skull) whose immobilisation will fix the local anatomical structures.

Away from such areas, there are still a number of sensitive regions whose irradiation should be avoided. These are (generally) regions that are especially prone to radiation damage, and/or linear organs where damage anywhere along the organ prevents the functioning of a large part of the organ. Examples Include the spinal cord, the optic nerve, the Intestines, and the like, Many of these are In anatomical regions that are easily moved by the patient, which would mean that a sensitive region could be placed into the beam.

One option would be to perform continuous or frequent CT scans, but each scan Involves delivering a radiation dose to the whole region. In general, it is desirable to limit this exposure for each patient, so if (or to the extent that) this can be avoided, It would be advantageous to do so.

The present invention seeks to combine confidence as to the correct positioning of the patient with the limitation of the number of CT scans that need to be delivered. The position of the patient's treatment volume is certain at the CBCT scan or directly thereafter, whereas It becomes more and more uncertain as time passes, i.e. the probability of movement of treatment volume Increases with time. In essence, the invention Is applicable to radiotherapy systems that deliver that fraction's dose in a series of individual doses, and provides that an initial CT scan Is performed to determine the patient's position, after which the individual doses that are directed to regions within a preset distance of a sensitive structure are then performed first, while individual doses directed to regions that are more remote from a sensitive structure are performed later, In this way, the doses for which the patient position is most critical are performed while the level of confidence in the patient position is at its highest, whereas later in the fraction when the patient position is less certain, the sub-doses whose exact positioning is less critical are delivered,

In a particular treatment, It may be that there are too many "near-sensitive" doses to deliver these within a reasonable time after the initial CT scan. Thus, in a preferred form of the invention, there is a post-CT time limit after which a "near-sensitive" dose may not be delivered, and if all the "near-sensitive" doses cannot be delivered within this time then a further CT scan or scans are scheduled for later in the fraction, with the remaining "near-sensitive" doses being delivered within the time limit after that CT scan, A suitable time limit could be 15 minutes, such as between 8 and 20 minutes, but will depend on the localization of the target and on the patient and will thus need to be assessed by a clinician.

The radiotherapy apparatus can be arranged to allow a user (such as a cliniclan) to highlight the critical Individual doses, i.e. those directed to a region within a preset distance of a sensitive structure or could calculate as a sensitivity analysis those doses that will contribute most to the sensitive region dose if they are moved. Alternatively, the user could highlight the sensitive structures and the apparatus could classify those Individual doses that are within a certain distance. Many radiotherapy treatment planning systems include the facility to classify regions as being sensitive In order to take this Into account in planning the shapes and direction of delivery of the sub-doses, so this information may already be available to the system. As a further alternative, the system could allow a user to rank the Individual doses in order of sensitivity.

Typically, a radiotherapy delivery system has an associated treatment planning system that accepts the physical and mechanical (etc) constraints of the delivery system, the Intended dose distribution, the details of the patient's anatomy, a definition of the target region and any sensitive regions that need to be avoided, and produces a treatment plan comprising a set of Instructions for the delivery system as to the nature, shape and direction of the radiation beams to emit. The above can be an additional constraint for the treatment planning system to take Into account; once the treatment plan is finalised, an additional step can be carried out in either ranking the Individual doses or allowing them to be ranked and checking for compliance with the time limit,

### BRIEF DESCRIPTION OF THE DRAWINGS

An embodiment of the present invention will now be described by way of example, with reference to the accompanying figures in which;
Figures 1 and 2 show (respectively) a front and side view of a first example of a radiotherapy apparatus suitable for use in the present invention;
Figure 3 shows a side view of a second example of a radiotherapy apparatus suitable for use In the present invention; and
Figures 4(a) to 4(e) show in schematic form alternative treatment plans, illustrating the nature and benefits of the present invention.

### DETAILED DESCRIPTION OF THE EMBODIMENTS

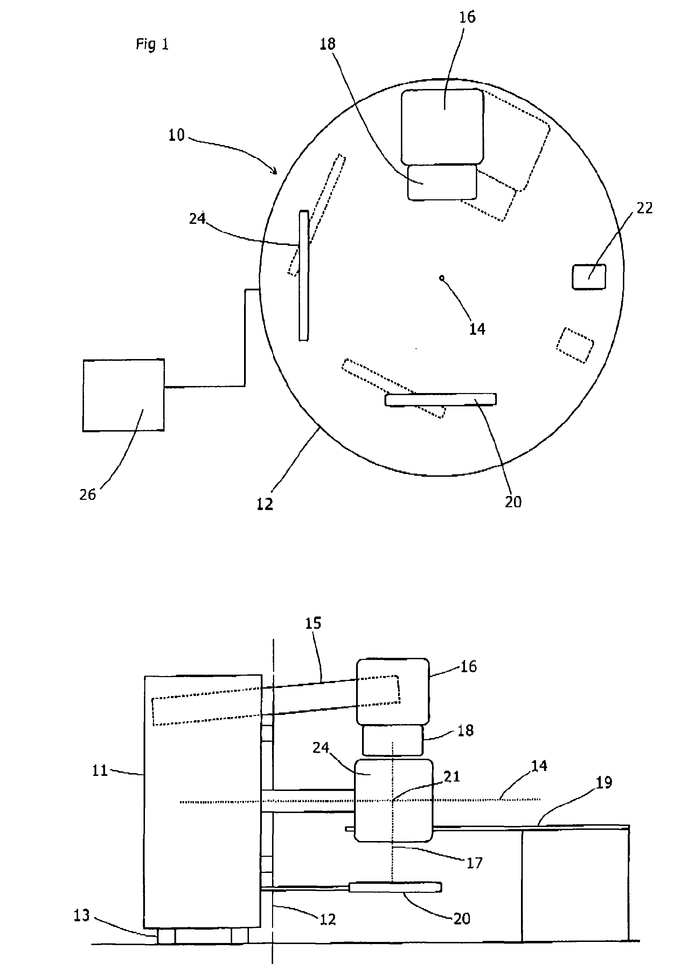

Referring to figures 1 & 2, a radiotherapy apparatus 10 comprises a rotateable faceplate 12 which is generally circular and mounted on a suitably substantial rotatable base 11. An arrangement of bearings and drives 13 allow the base 11 to rotate around a central horizontal axis 14 In a generally known manner.

A linear accelerator 15 is built into the base 11 and extends forward from the support at an off-centre position, ending in a source of megavoltage (MV) therapeutic X-radiation 16 directed radially inwardly towards the rotation axis 14. Collimation apparatus 18 is provided on the source 16, in a generally known manner so as to shape the beam as required. A patient can be supported on an adjustable table 19, positioned so that a target region is located approximately at the point of intersection of the rotational axis 14 and the centreline 17 of the beam emitted by the source 16 (a point known as the "isocentre" 21). The base 11 can then be rotated around the patient, allowing the source 16 to Irradiate the patient from a variety of directions. In this way, the dose to the target region can be maximised whilst minimising the dose to the surrounding healthy tissue.

A megavoltage imaging panel 20 is provided, supported from the base 11 diametrically opposite the MV source 16. This captures an image from the radiation emitted by the source 16 as attenuated by the patient and can provide useful information as to the Internal structure of the patient.

Many radiotherapy apparatus also include a diagnostic kV source 22. This is also mounted on the base 11, typically located 90 degrees away from the MV source 16. Collimation can be provided for this source, usually static in nature, In order to limit the beam to the desired aperture size, A corresponding flat panel two-dimensional kV Imaging panel 24 is also provided, diametrically opposite the kV source 22. These can be used to capture radiographs of the relevant region of the patient; a number of such images can be captured and combined to produce a computed-tomography (CT) scan using known techniques.

A control unit 26 serves to control the radiation sources 16, 22, the collimator 18, the flat panel Imagers 20, 24, the support 12, and other equipment that may be provided as required. This control is exercised in line with a "treatment plan" developed in advance based on knowledge of the patient anatomy, the tumour location, the properties of the beam produced by that source 16, the constraints of the collimator 18 and the other items of equipment (such as the range of movement of the table 19) etc. Typically, the treatment plan is created overnight prior to the treatment by dedicated treatment planning computers which accept these constraints and use known software and algorithms to generate a treatment plan.

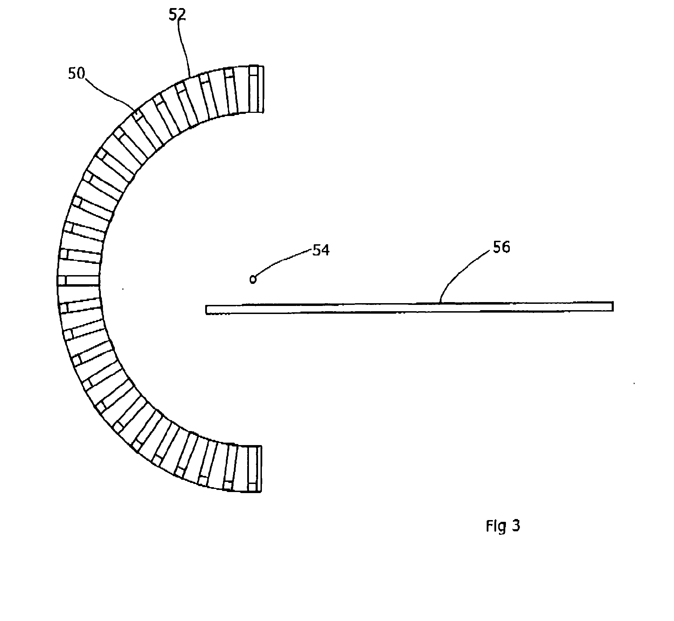

An alternative delivery system is illustrated in figure 3, showing a multiple-source radiotherapy apparatus in the form of a Leksell Gamma Knife™. This typically contains 192 Cobalt-60 sources 50, each of approximately 30 curies (1.1 TBq), each placed on a sector arrangement of eight sectors in a semi spherical configuration in a heavily shielded assembly 52. Each source is collimated by the assembly 52 and aims gamma radiation at a target point 54. Collimators (not shown) allow the sources to be exposed during treatment or concealed while the patient Is being positioned by moving the sectors to an off position. All collimators aim at the same position In space, the target.

A patient can be supported on a table 56 so as to locate the tumour at the target point 54. Each Individual beam Is of relatively low intensity, so the radiation has little effect on Intervening brain tissue and Is concentrated only at the tumor itself. Once the patient is positioned, the collimators are opened for the required period of time in order to deliver an individual dose. The sources are then put to an off position while moving the patient to the next position where dose Is to be delivered. Normally all sources are delivering dose simultaneously, though some can be blocked allowing only a subset of the sources to deliver a dose for one or more patient positions.

Historically, the Gamma Knife has been used for cranial tumours, which have a stable location relative to hard structures around them such as the skull. Accordingly, It Is common to fixate the patient's skull using known frames that can be securely attached to the patient support. Movement of the tumour during a fraction can therefore be discounted. However, It Is being proposed that Gamma Knife systems be used for treating tumours In the neck and upper shoulders, where there is no suitable fixed structure that has a rigid relationship with the tumour. Also, these areas include the spinal cord, which must not be accidentally irradiated.

Whatever form of delivery method is used, it Is necessary to develop a treatment plan setting out the doses of radiation that are to be delivered, which achieves the prescribed dose while remaining within the various constraints. The patient-Imposed constraints on the treatment plan are generally derived from classifying (or "segmenting") the volume of the patient into one of three categories:
a) tumour tissue, into which the prescribed dose is to be delivered. This dose may be uniform, or may consist of different doses in different areas. Generally, a margin of healthy tissue around the actual tumour volume is also Included within this category, to allow for minor movement of the tumour and/or patient during treatment and for scanning tolerances,
b) Ordinary tissue such as muscle, skin, bone and resilient organs such as the liver, Into which the delivered dose is to be limited so far as possible, and
c) Sensitive volumes such as eyes, the optic nerve, the spinal cord etc, into which the delivered therapeutic dose is to be substantially zero.

Other constraints arise from the nature of the delivery method and (obviously) the dose distribution to be achieved. These constraints are then provided to the treatment planning computer, which produces a treatment plan consisting of instructions for the radiotherapy apparatus.

The treatment plans for some systems consist of a series of Individual doses that are to be delivered to the patient. The main alternative is, for example, a single dose that Is delivered from a source that moves and/or adjusts Its collimation during the fraction (such as VMAT-based techniques). In either case, the treatment will take a period of time to deliver, and is created on the assumption that the patient remains stationary throughout. This assumption may not be valid, depending on the time required and the particular patient. Generally, up-to-date radiotherapy apparatus comprises a CBCT functionality together with the irradiation equipment. This allows a CBCT scan to be taken at the start of the fraction In order to locate the patient (by corresponding compensatory adjustment of the patient support), and either the CBCT scan will be repeated after intervals in order to update the position of the patient (and, if necessary, correct It) or some form of fixation device will be used to Immobilise the patient, The latter can be uncomfortable, and the former Is In principle undesirable unless absolutely necessary, as a CBCT scan involves delivering a non-trivial dose of radiation to the entire volume, Some systems provide a conventional CT scanner which is Incorporated within the radiotherapy apparatus; the following discussion Is also valid for a such an arrangement, using a CBCT scan instead.

In treatment plans of the former type, i.e. those consist of a series of Individual doses, the present invention allows fewer CBCT scans to be used whilst still having the necessary degree of confidence. This is based on the realisation that the likellhood of a certain amount of movement by the patient Increases with time, therefore those individual doses for which the patient positioning Is most critical should be scheduled earliest after a CBCT scan, and those for which the patient position is not so critical can be scheduled later. Thus, by classifying an individual dose according to Its proximity to a sensitive structure, a decision can be made as to where in the sequence of that fraction the individual dose should be scheduled.

That classification can be a binary decision, i.e. that the individual dose is within a certain distance of a critical structure or that It Is not. Alternatively, the classification can be a system describing which range of distances the individual dose falls into, such as "very close", "proximate" or "dlstant", which may be coded as "1", "2" or "3". In a further alternative, the closest approach distance of the dose to a sensitive structure could be employed to provide an analogue scale.

Another way of classifying individual doses could be to let the treatment planning system perform a sensitivity analysis for each of the doses to be delivered ("shots''), whereby the effect of varying a shot position is determined automatically and the increase of dose to the sensitive tissue is calculated. This allows the relative Importance of positional accuracy to be determined for each of the shots, In terms of the potential sensitivity of a positional error In a specific shot, The shot at the position with the largest contribution to dose In sensitive tissue when that shot Is moved is thereafter selected to be delivered first, as it presents the highest risk If there should be a movement or positioning error. More generally, the shots can be ranked into an order for delivery by a suitable algorithm. For example, if there are six shots ranked in decreasing order of sensitivity as shots 1-6, these could be delivered in the order [CBCT scan], 1, 2, 3, 4, 5, 6, so that the most positionally sensitive are placed first. If there Is insufficient time after the scan to deliver these, then al alternative order such as [CBCT scan], 1, 3, 5, [CBCT scan], 2, 4, 6 can be adopted (etc).

The classification could be done automatically by the treatment planning computer, based on its knowledge of the individual dose locations and the locations of the sensitive structures, Alternatively, a suitably skilled user could classify the individual doses. Some classification systems will of course be better suited to automation, and some to user selection.

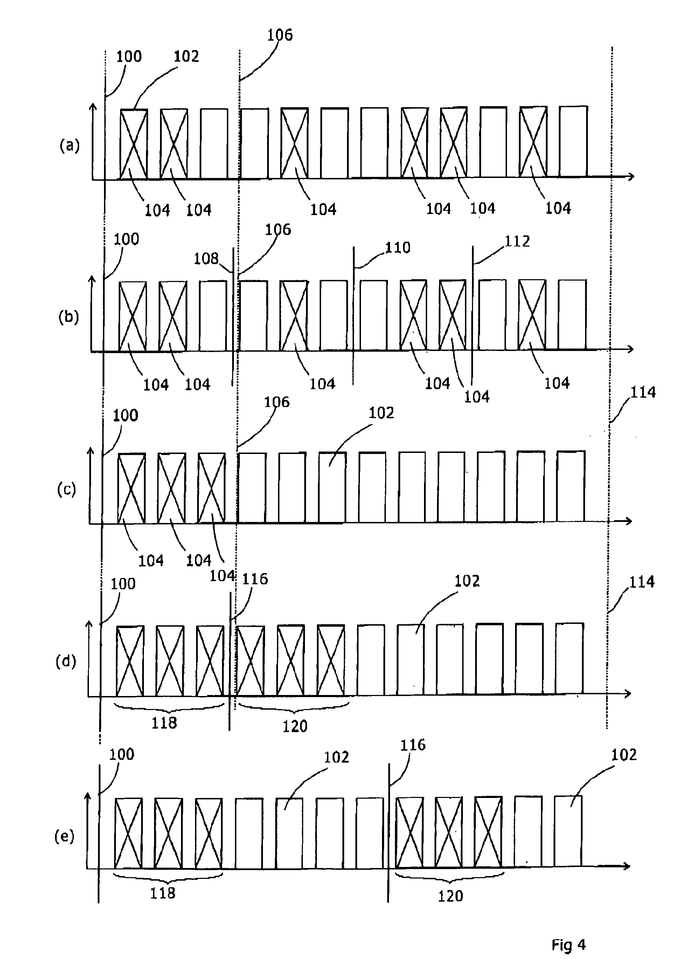

Figure 4 shows the effect of implementing a binary classification system on a simple treatment plan consisting of 12 individual doses, of which 6 are near to a sensitive structure. Figure 4(a) shows a known arrangement. An initial cone-beam CT scan 100 is performed, followed by a series of individual doses 102. Highlighted doses 104 are proximate a sensitive structure, and are distributed throughout the treatment fraction. If we assume (for the sake of illustration) that after a period of time equivalent to three Individual doses, there is a significant risk that the patient has moved an amount that Is enough to place a sensitive structure at risk, then this means that many of the highlighted doses are outside this safe region 106. On this basis, the plan shown In figure 4(a) exhibits an unacceptable risk in that the later doses will be delivered without adequate confidence In the location of the patient. Therefore, to assure safety, three additional CBCT scans 108, 110 and 112 must be performed as shown In figure 4(b) so that all the highlighted doses are within this safe period of time.

Of course, the particular period of time chosen may be different, and individual doses may be of different exposure times, meaning that the chosen period may not correspond to an exact number of individual doses. However, the principle can be Illustrated in this way.

Figure 4(c) shows a first embodiment of the invention, The highlighted doses 104 are all grouped together at the start of the treatment, and are followed by the remaining doses 102. Thus, those doses 104 that are proximate to sensitive areas of the patient are delivered within the safe region 106 after the initial CT scan 100, The other doses are delivered within a longer period 114 during which it is safe to assume that the patient has not moved so far as to render those doses unsafe, although the patient may have moved too far for a near-sensitive dose to be considered unsafe.

In figure 4(c), there are of course fewer near-sensitive doses 104 than in the preceding examples of figures 4(a) and 4(b), reflecting the fact that the safe period after the initial CT scan 100 is (in this case) only sufficient to allow three individual doses to be delivered. To deliver more, an additional CT scan 116 needs to be performed into order to "re-set" the patient position, establishing this with confidence and allowing further near-sensitive doses to be delivered. This can be done as In figures 4(d) or 4(e).

Figure 4(d) shows the additional CT scan 116 being performed Immediately after the expiry of the safe period 106. This means that a first group 118 of near-sensitive doses can be delivered immediately after the Initial CT scan 100, followed by the additional CT scan 116, followed by a second group 120 of near-sensitive doses, followed by the remaining individual doses 102.

Figure 4(e) shows an alternative Implementation of the invention, in which the first group 118 of near-sensitive doses is again delivered immediately after the initial CT scan 100, but is followed by some of the "ordinary" doses 102. These are followed by the additional CT scan 116, which is then followed by a second group 120 of near-sensitive doses, followed by the remaining individual doses 102. This allows the additional CT scan (or scans) 116 to be distributed more evenly through the fraction, meaning that all doses are delivered more promptly after a CT scan. Note that In figure 4(e), the longest period between a CT scan and an Individual dose is 7 doses, as opposed to 9 doses In figure 4(d). Both figures 4(d) and 4(e) require only two CT scans, however, compared to four scans In figure 4(b).

Table 1 shows another example, In this example, individual doses or "shots" are ranked according to their level of criticality by a clinician on a scale of 1 to 3. These are then scheduled for delivery in order, with the highest ranked (level 1) first. This reveals that there is insufficient time to deliver all the ranked shots within a safe period after the Initial cone-beam CT scan, so they are divided into two groups with an additional CT scan prior to the second group. To further optimise the delivery, the category "1" shots are divided into two sub-groups and each sub-group Is placed at the start of Its respective group, thereby ensuring that the shots with the highest level of criticality are delivered soonest after a CT scan, and so one for the level "2" shots. The level "3" shots are then distributed among the groups so as to remain within the safe periods after a CT scan and minimise the times spent on each group. The result of this optimisatlon Is set out in table 1.

**Table 1**

| | Criticality | Time (minutes) |
|---|---|---|
| | ---- CBCT ---- | |
| Critical shots | 1 | 1.8 |
| | 1 | 1.3 |
| | 2 | 1.4 |
| | 3 | 1.8 |
| | 3 | 1.2 |
| | ---- CBCT ---- | |
| | 1 | 2 |
| | 2 | 2.5 |
| | 2 | 1.4 |
| Normal Shots | - | 3 |
| | - | 5 |
| | - | 8 |

Thus, the present Invention allows individual doses within a treatment fraction to be delivered with greater confidence as to the patient position, without having to Incur unnecessary CBCT-related doses. This Is able to improve the accuracy to target to probably sub-millimeter In respect of the critical shots, thereby making a single session possible when using the Elekta LGK PERFEXION system, without requiring an IFMM (intra-fraction motion management) solution.

It will of course be understood that many variations may be made to the above-described embodiment without departing from the scope of the present invention.

## Claims

1. A radiotherapy treatment planning apparatus, adapted to receive details of constraints relating to a radiotherapy treatment and create a treatment plan for a radiotherapy system,
the constraints including location information as to at least one target region within the patient that is to be irradiated and at least one sensitive region within the patient, irradiation of which is to be avoided;
wherein the treatment plan includes details of a series of individual doses to be delivered to different locations within the patient during a treatment fraction, and
wherein the treatment planning apparatus is arranged to deliver individual doses that are directed to a region proximate a sensitive structure earlier in the series than individual doses directed to a region not proximate a sensitive structure,

2. A radiotherapy treatment planning apparatus according to claim 1 wherein the treatment planning apparatus is adapted, in response to the classification of each individual dose according to its proximity to a sensitive structure, to schedule each individual dose in the series of doses.

3. A radiotherapy treatment planning apparatus according to claim 1 or claim 2 wherein a region is proximate a sensitive structure if it is within a preset distance of a sensitive structure.

4. A radiotherapy treatment planning apparatus according to claim 1 or claim 2 wherein a region is proximate a sensitive structure if it is marked by a user as being proximate a sensitive structure.

5. A radiotherapy treatment planning apparatus according to any of claims 1 to 3 wherein the treatment plan includes details of one or more CT scans to be performed within the fraction.

6. A radiotherapy treatment planning apparatus according to claim 5, wherein the treatment plan includes, at least:
a first CT scan,
subsequently thereto, a plurality of individual doses that are directed to a region proximate a sensitive structure,
subsequently thereto, a further CT scan,
subsequently thereto, at least one individual dose that is directed to a region proximate a sensitive structure, and
at least one individual dose directed to a region not proximate a sensitive structure, immediately after one of the aforesaid individual doses directed to a region proximate a sensitive structure.

7. A radiotherapy treatment planning apparatus according to claim 5 or claim 6 in which the constraints include a defined time limit subsequent to a CT scan, after which a individual dose that is directed to a region proximate a sensitive structure may not be delivered.

8. A radiotherapy treatment planning apparatus according to claim 7 arranged to detect if all the individual doses that are directed to a region proximate a sensitive structure cannot be delivered within the defined time limit and, if so, insert a further CT scan into the treatment plan and schedule at least some of the remaining individual doses directed to a region proximate a sensitive structure after that CT scan.

9. A radiotherapy treatment planning apparatus according to claim 7 or claim 8 in which the time limit is between 8 and 20 minutes.

10. A radiotherapy treatment planning apparatus according to claim 7 or claim 8 in which the time limit is approximately 10 minutes.

## Patentansprüche

1. Strahlentherapie-Planungsvorrichtung, die ausgelegt ist, Details über Einschränkungen in Bezug auf eine Strahlentherapie zu empfangen und einen Behandlungsplan für ein Strahlentherapiesystem zu erstellen,
wobei die Einschränkungen Lokalisationsangaben bezüglich zumindest einer Zielregion in dem Patienten, die bestrahlt werden soll, und zumindest einer empfindlichen Region in dem Patienten, deren Bestrahlung zu vermeiden ist, umfassen;
worin der Behandlungsplan Details über eine Reihe von individuellen Dosierungen aufweist, die an verschiedene Stellen in dem Patienten während einer Therapiefraktion verabreicht werden sollen, und
worin die Therapieplanungsvorrichtung angeordnet ist, individuelle Dosierungen, die auf eine Region neben einer empfindlichen Struktur gerichtet sind, früher in der Reihe zu verabreichen als individuelle Dosierungen, die auf eine Region gerichtet sind, die nicht neben einer empfindlichen Struktur liegt.

2. Strahlentherapie-Planungsvorrichtung nach Anspruch 1, worin die Therapieplanungsvorrichtung ausgelegt ist, als Reaktion auf die Klassifizierung jeder individuellen Dosis gemäß ihrer Nähe zu einer empfindlichen Struktur, jede individuelle Dosis in der Dosisreihe zu planen.

3. Strahlentherapie-Planungsvorrichtung nach Anspruch 1 oder Anspruch 2, worin eine Region neben einer empfindlichen Struktur liegt, wenn sie innerhalb eines vorbestimmten Abstands von einer empfindlichen Struktur liegt.

4. Strahlentherapie-Planungsvorrichtung nach Anspruch 1 oder Anspruch 2, worin eine Region neben einer empfindlichen Struktur liegt, wen sie von einem Anwender als neben einer empfindlichen Struktur liegend markiert wird.

5. Strahlentherapie-Planungsvorrichtung nach einem der Ansprüche 1 bis 3, worin der Behandlungsplan Details über einen oder mehrere CT-Scans aufweist, die innerhalb der Fraktion durchgeführt werden sollen.

6. Strahlentherapie-Planungsvorrichtung nach Anspruch 5, worin der Behandlungsplan zumindest Folgendes umfasst:
einen ersten CT-Scan,
im Anschluss daran eine Vielzahl von individuellen Dosierungen, die auf eine Region neben einer empfindlichen Struktur gerichtet sind,
im Anschluss daran einen weiteren CT-Scan,
im Anschluss daran zumindest eine individuelle Dosis, die auf eine Region neben einer empfindlichen Struktur gerichtet ist, und
zumindest eine individuelle Dosis, die auf eine Region gerichtet ist, die nicht neben einer empfindlichen Struktur liegt, unmittelbar nach einer der oben erwähnten individuellen Dosierungen, die auf eine Region neben einer empfindlichen Struktur gerichtet sind.

7. Strahlentherapie-Planungsvorrichtung nach Anspruch 5 oder Anspruch 6, in der die Einschränkungen eine definierte Zeitbegrenzung nach einem CT-Scan umfassen, nach der eine individuelle Dosis, die auf eine Region neben einer empfindlichen Struktur gerichtet ist, nicht verabreicht werden darf.

8. Strahlentherapie-Planungsvorrichtung nach Anspruch 7, ausgelegt zum Nachweis, ob alle individuellen Dosierungen, die auf eine Region neben einer empfindlichen Struktur gerichtet sind, innerhalb der definierten Zeitbegrenzung nicht verabreicht werden dürfen, und, falls ja, zum Einfügen eines weiteren CT-Scans in den Therapieplan und Planen zumindest einiger der verbleibenden individuellen Dosen, die auf eine Region neben einer empfindlichen Struktur gerichtet sind, nach diesem CT-Scan.

9. Strahlentherapie-Planungsvorrichtung nach Anspruch 7 oder Anspruch 8, wobei die Zeitbegrenzung zwischen 8 und 20 Minuten beträgt.

10. Strahlentherapie-Planungsvorrichtung nach Anspruch 7 oder Anspruch 8, wobei die Zeitbegrenzung zwischen 10 Minuten beträgt.

## Revendications

1. Appareil de planification de traitement de radiothérapie, adapté pour recevoir les détails de contraintes relatives à un traitement de radiothérapie et créer un plan de traitement pour un système de radiothérapie,
les contraintes comportant des informations de position quant à au moins une région cible à l'intérieur du corps du patient qui doit être irradiée et au moins une région sensible à l'intérieur du corps du patient dont l'irradiation doit être évitée ;
le plan de traitement comportant les détails d'une série de doses individuelles à délivrer à différentes positions à l'intérieur du corps du patient pendant une fraction de traitement, et
l'appareil de planification de traitement étant configuré pour délivrer des doses individuelles qui sont dirigées vers une région proche d'une structure sensible plus tôt dans la série que les doses individuelles dirigées vers une région éloignée d'une structure sensible.

2. Appareil de planification de traitement de radiothérapie selon la revendication 1, l'appareil de planification de traitement étant adapté, en réponse au classement de chaque dose individuelle en fonction de sa proximité d'une structure sensible, pour programmer chaque dose individuelle dans la série de doses.

3. Appareil de planification de traitement de radiothérapie selon la revendication 1 ou la revendication 2 dans lequel une région est proche d'une structure sensible si elle se situe à moins d'une distance prédéfinie d'une structure sensible.

4. Appareil de planification de traitement de radiothérapie selon la revendication 1 ou la revendication 2 dans lequel une région est proche d'une structure sensible si elle est marquée par un utilisateur comme étant proche d'une structure sensible.

5. Appareil de planification de traitement de radiothérapie selon l'une quelconque des revendications 1 à 3 dans lequel le plan de traitement comporte les détails d'un ou plusieurs tomodensitogrammes à effectuer à l'intérieur de la fraction.

6. Appareil de planification de traitement de radiothérapie selon la revendication 5, dans lequel le plan de traitement comporte au moins :
un premier tomodensitogramme,
après celui-ci, une pluralité de doses individuelles qui sont dirigées vers une région proche d'une structure sensible,
après celles-ci, un autre tomodensitogramme,
après celui-ci, au moins une dose individuelle qui est dirigée vers une région proche d'une structure sensible, et
au moins une dose individuelle dirigée vers une région éloignée d'une structure sensible, juste après une des doses individuelles précitées dirigées vers une région proche d'une structure sensible.

7. Appareil de planification de traitement de radiothérapie selon la revendication 5 ou la revendication 6 dans lequel les contraintes comportent une limite de temps définie après à un tomodensitogramme, après laquelle une dose individuelle qui est dirigée vers une région proche d'une structure sensible ne peut pas être délivrée.

8. Appareil de planification de traitement de radiothérapie selon la revendication 7 configuré pour détecter si toutes les doses individuelles qui sont dirigées vers une région proche d'une structure sensible ne peuvent pas être délivrées dans la limite de temps définie et, le cas échéant, insérer un autre tomodensitogramme dans le plan de traitement et programmer au moins certaines des doses individuelles restantes dirigées vers une région proche d'une structure sensible après ce tomodensitogramme.

9. Appareil de planification de traitement de radiothérapie selon la revendication 7 ou la revendication 8 dans lequel la limite de temps se situe entre 8 et 20 minutes.

10. Appareil de planification de traitement de radiothérapie selon la revendication 7 ou la revendication 8 dans lequel la limite de temps est d'environ 10 minutes.
